# DEMANDE DE BREVET EUROPEEN

(11) **EP 3 591 763 A1**
(43) Date de publication de la demande: **08.01.2020**
(21) Numéro de dépôt: 19184141.0
(22) Date de dépôt: 03.07.2019
(51) Int. Cl.: H01R 4/02, A61N 1/04, H01R 4/20, H01R 43/02, H01R 43/05, H01R 43/28

(54) **PROCEDE DE CONNEXION POUR CONNECTER UN MICRO-CONDUCTEUR ISOLE**

(30) Priorité: 06.07.2018 FR 1856248
(71) Demandeur: Sorin CRM SAS, 92140 Clamart (FR)
(72) Inventeur: JULIEN, Etienne, 92310 Sèvres (FR); SHAN, Nicolas, 92160 Antony (FR); KROISS, Daniel, 38330 Saint Ismier (FR)
(74) Mandataire: Grünecker Patent- und Rechtsanwälte PartG mbB

(57) **Abrégé**

La présente invention se rapporte à un procédé pour connecter un toron (2) d'un câble multitoron (1) à un élément conducteur (5), en particulier à une électrode (5), ainsi qu'à un tel câble multi-toron (1).

## Description

La présente invention se rapporte à un procédé pour connecter un micro-conducteur isolé d'un câble multi-micro conducteurs, notamment à une électrode d'un dispositif médical implantable.

En particulier, la présente invention concerne la connexion d'un microcâble isolé, aussi appelé toron, d'un microcâble multi-toron à une électrode de stimulation, tel que chaque toron individuel est isolé électriquement des autres torons par une gaine isolante, en particulier par une gaine en polymère.

Un assemblage composite d'un tel microcâble présente des difficultés intrinsèques pour se connecter à une électrode, par exemple dans le cadre de la fabrication d'un implant, liées à la taille des brins conducteurs, c'est-à-dire des torons, à relier à l'électrode (de l'ordre de 100µm), mais aussi liées à l'épaisseur des isolants très fine - permettant de garantir la faible taille de l'ensemble, et enfin aux difficultés liées à la proximité des conducteurs les uns aux autres et de la fiabilité dans le temps du contact, en milieu in-vivo.

EP 2 719 422 B1 décrit une approche permettant de réaliser une ablation de l'isolant localement, et de déposer une colle conductrice. Cependant, la colle conductrice, utilisée dans un environnement biologique, est susceptible d'être biodégradée, ce qui peut affecter le contact électrique. Une autre difficulté provient de l'étape consistant à déposer dans une cavité de 50µm-80µm une quantité reproductible de colle conductrice.

EP 2 674 190 B1 décrit une fabrication additive, par ajout successif de couches de matière métallique, permettant de se contacter au micro câble avec une électrode intégrant un mécanisme de contacteur. Ce type de mécanisme a cependant des inconvénients au niveau de la précision du contact et sa stabilité, car celui-ci dépendra de la tolérance des pièces mécaniques en contact, ainsi que de l'épaisseur d'isolant. D'autre part, cette technique ne semble pas adaptée à un câble multi-toron.

L'objet de l'invention est donc de fournir un procédé de connexion qui puisse améliorer un contact électrique de faible résistance, robuste et durable dans le temps dans une structure de l'ordre du micron - surtout en milieu biologique sous contrainte mécaniques entre un micro-conducteur (toron) isolé et une électrode.

L'objet de la présente invention est atteint par un procédé pour connecter un toron d'un câble multi-toron à un élément conducteur, en particulier à une électrode, comprenant les étapes de a) découper un toron du câble multi-toron afin de former deux extrémités libres au niveau de la coupe du toron; b) soulever au moins une des extrémités libres par rapport au reste du câble multi-toron ; c) dénuder au moins partiellement l'extrémité du toron soulevée; d) placer un élément conducteur autour du câble multi-toron de manière à recouvrir partiellement l'extrémité du toron soulevée et dénudée ; e) connecter au moins une portion de l'extrémité dénudée du toron à l'élément conducteur.

L'extraction d'un des torons du microcâble rend possible d'extraire l'ensemble du revêtement isolant dans la zone des soudures et ainsi éviter des difficultés liées à la présence de polymère dans une soudure, notamment une soudure laser, nuisant à sa qualité, en particulier à une échelle de l'ordre du micron.

Ce procédé, en plus de permettre des conditions de soudure fiable, permet aussi d'améliorer la robustesse à long terme de la jonction électrique entre le toron isolé du reste du microcâble et l'électrode.

La présente invention peut être davantage améliorée grâce aux modes de réalisation suivants.

Selon une autre réalisation de l'invention, l'étape b) peut comprendre : recouvrir l'autre des extrémités libres du toron en introduisant un manchon isolant autour du câble. Ce manchon isolant permet d'éviter que l'autre extrémité ne s'échappe ou ne se soulève sur reste du câble.

Selon une autre réalisation de l'invention, l'étape b) peut également comprendre la pose d'un deuxième manchon ou/et d'une colle polymère autour du câble multi-toron afin de contrôler la longueur (L) de l'extrémité du toron soulevée par rapport au reste du câble multi-toron. Ce deuxième manchon permet aussi, selon sa position, de s'assurer de la longueur de l'extrémité du toron qui a été soulevée et de maintenir cette longueur grâce au deuxième manchon.

Selon une autre réalisation de l'invention, l'étape a) et c) peuvent comprendre une découpe ou/et ablation laser ou l'utilisation d'un dispositif mécanique de découpe. Ainsi, ce procédé est apte à être réalisé à partir de technologies maîtrisées. De plus, selon le procédé de la présente invention, la soudure peut être réalisée avec contrôle visuel ce qui améliore davantage la fiabilité de la soudure.

L'objet de la présente invention est aussi atteint par un procédé pour connecter un toron d'un câble multi-toron à un élément conducteur, en particulier à une électrode, comprenant les étapes de a) découper un toron du câble multi-toron afin de former deux extrémités libres au niveau de la coupe du toron; b) soulever au moins une des extrémités libres par rapport au reste du câble multi-toron; c) placer un hypotube métallique autour de l'extrémité du toron soulevée de manière à ce que l'hypotube soit électriquement connecté à l'extrémité du toron; d) placer un élément conducteur autour du câble multi-toron de manière à recouvrir partiellement l'hypotube métallique; e) connecter au moins une portion de l'hypotube métallique à l'élément conducteur.

Ce procédé, grâce à l'utilisation d'hypotube métallique offre des conditions de soudure fiable, et permet aussi d'améliorer la robustesse à long terme de la jonction électrique entre le toron isolé du reste du microcâble et l'électrode. En effet, la soudure est réalisée au niveau de l'hypotube ce qui permet d'éviter des difficultés liées à la présence de polymère dans une soudure, notamment dans une soudure laser.

Les deux procédés de la présente invention décrits ci-dessus peuvent davantage être améliorés grâce aux modes de réalisation suivants.

Selon une autre réalisation de l'invention, la connection à l'étape e) peut être réalisée par soudure laser, par sertissage ou par soudure électrique.

Selon une autre réalisation de l'invention, chaque procédé peut comprendre une étape f) pour poser un gainage en polymère afin de revêtir le câble multi-toron et l'électrode soudée au câble. Cette étape permet d'ajouter de manière avantageuse une barrière isolante au microcâble auquel est soudée l'électrode.

Selon une autre réalisation de l'invention, l'élément conducteur peut être une électrode. De manière avantageuse, l'électrode peut être en forme de bague telle que la bague puisse comprendre un trou central ou une fente configuré pour réaliser la soudure à l'étape e).

Dans une autre réalisation de l'invention, l'électrode peut être en forme de bague telle que la bague puisse comprendre une cavité interne adaptée à la dimension de l'extrémité du toron soulevé. Cette cavité interne permet de laisser l'espace nécessaire à l'extrémité du toron à souder et améliore ainsi davantage la surface de contact électrique.

Dans une autre réalisation de l'invention, le diamètre d'un toron - en prenant en considération la partie conductrice et la partie isolante du toron - peut être compris entre 10 µm et 200µm; le diamètre du microcâble multi-toron, c'est-à-dire le diamètre du câble entier, peut être inférieur à 2 French (soit 0,66mm) et peut être réalisé à partir de matériaux biocompatibles. Pour la partie isolante du toron, des matériaux tels que les polymères fluorés, notamment le PTFE (polytétrafluoroéthylène), le FEP (propylène perfluoré), le PFA (résine de copolymère perfluoroalkoxy), le THV (tétrafluoroéthylène, hexafluoropropylène, fluorure de vinylidène), le PVDF (polyfluorure de vinylidène), l'EFEP (éthylène propylène éthylène fluoré), ou l'ETFE (éthylène tétrafluoroéthylène). De plus, les polyuréthannes (PU), polyesters (PET), polyamides (PA), polycarbonates (PC), polyimides, polymères fluorés, le polyéther-éther-cétone (PEEK), le poly-p-xylylène (parylène), ou le polyméthacrylate de méthyle (PMM) peuvent également être utilisés tels que mentionnés dans EP3058983.

La partie conductrice du coeur des torons peut être réalisée par des matériaux tels que les aciers inox, les alliages de cobalt, le titane, l'alliage NiTi (nitinol), le tantale (Ta), le tungstène (W), l'iridium (Ir), le platine (Pt), l'or (Au) et leurs alliages tels que mentionnés dans EP2581107. Ainsi, le microcâble est adapté pour être utilisé en combinaison avec des dispositifs implantables.

La présente invention est également atteinte par un microcâble multi-toron comprenant au moins un élément conducteur tel qu'au moins une extrémité d'un toron du microcâble est partiellement dénudée de manière à ce que la portion partiellement dénudée du toron est connectée, en particulier par soudure, sertissage ou soudure électrique, à l'élément conducteur.

Ainsi, la surface de contact entre le toron isolé et l'élément conducteur est améliorée. Dans une autre réalisation de l'invention, le microcâble peut comprendre un manchon isolant positionné autour du câble et sur lequel l'extrémité du toron est positionnée. Ce manchon isolant permet d'éviter que l'autre extrémité ne s'échappe ou ne se soulève sur reste du câble.

De manière alternative, la présente invention est également atteinte par un microcâble multitoron comprenant au moins un élément conducteur et un hypotube métallique tel qu'au moins une extrémité d'un toron du microcâble est au moins partiellement en contact électrique avec l'hypotube, de manière à ce que l'hypotube est connecté, en particulier par soudure, sertissage ou soudure électrique, à l'élément conducteur.

La présente invention, relative à un microcâble multi-toron, peut être davantage améliorée grâce aux modes de réalisation suivants.

Selon une autre réalisation de l'invention, l'élément conducteur peut être une électrode en forme de bague telle que la bague comprend un trou central ou une fente configuré pour réaliser la soudure avec la portion d'une extrémité du toron.

Selon une autre réalisation de l'invention, l'élément conducteur peut être une électrode en forme de bague telle que la bague comprend une cavité interne adaptée à la dimension de l'extrémité du toron arrangée partiellement au-dessus du manchon isolant. Cette cavité interne permet de laisser l'espace nécessaire à l'extrémité du toron à souder et améliore ainsi davantage la surface de contact électrique.

Selon une autre réalisation de l'invention, le diamètre d'un toron - en prenant en considération la partie conductrice et la partie isolante du toron - peut être compris entre 10µm 200µm ; le diamètre du microcâble multi-toron, c'est-à-dire le diamètre du câble entier, peut être inférieur à 2 French (soit 0,66mm) et peut être réalisé à partir de matériaux biocompatibles. Pour la partie isolante du toron, des matériaux tels que les polymèresn fluorés, notamment le PTFE (polytétrafluoroéthylène), le FEP (propylène perfluoré), le PFA (résine de copolymère perfluoroalkoxy), le THV (tétrafluoroéthylène, hexafluoropropylène, fluorure de vinylidène), le PVDF (polyfluorure de vinylidène), l'EFEP (éthylène propylène éthylène fluoré), ou l'ETFE (éthylène tétrafluoroéthylène). De plus, les polyuréthannes (PU), polyesters (PET), polyamides (PA), polycarbonates (PC), polyimides, polymères fluorés, le polyéther-éther-cétone (PEEK), le poly-p-xylylène (parylène), ou le polyméthacrylate de méthyle (PMM) peuvent également être utilisés tels que mentionnés dans EP3058983.

La partie conductrice du coeur des torons peut être réalisées par des matériaux tels que les aciers inox, les alliages de cobalt, le titane, l'alliage NiTi (nitinol), le tantale (Ta), le tungstène (W), l'iridium (Ir), le platine (Pt), l'or (Au) et leurs alliages tels que mentionnés dans EP2581107. Ainsi, le microcâble est adapté pour être utilisé en combinaison avec des dispositifs implantables.

La présente invention, relative à au procédé ou au microcâble multi-toron, peut être davantage améliorée selon les variantes suivantes.

Selon une variante de l'invention, la soudure peut être réalisée au niveau des deux extrémités libres du même toron afin de garantir une redondance de la liaison.

Dans encore une autre variante, deux torons en opposition peuvent être découpés, soulevés et partiellement extraits du reste du câble et soudés des deux côtés.

Enfin, dans une autre variante, la séparation des brins d'un même câble en un nombre n de sous-câbles ou fils permet de faire de la redondance de soudure et ainsi d'améliorer la fiabilité du contact électrique avec l'élément conducteur par augmentation du nombre de soudures. Notamment dans le cas où une des soudures se casse, les n-1 soudures restantes permettent d'assurer le contact électrique. Ainsi, la redondance de soudure améliore la robustesse du contact électrique.

L'invention et ses avantages seront expliqués plus en détail dans la suite au moyen de modes de réalisation préférés et en s'appuyant notamment sur les figures d'accompagnement suivantes, dans lesquelles :
la figure 1 illustre schématiquement un microcâble à l'étape a) du procédé selon la présente invention;
les figures 2 et 3 illustrent schématiquement un microcâble à l'étape b) du procédé selon la présente invention;
les figures 4 et 5 illustrent schématiquement un microcâble aux étapes c) et d) du procédé selon la présente invention;
la figure 6 illustre schématiquement un microcâble à l'étape e) du procédé selon la présente invention;
la figure 7 illustre schématiquement un microcâble à l'étape f) du procédé selon la présente invention;
les figures 8a, 8b, 9 et 10 illustrent schématiquement des réalisations alternatives d'un élément conducteur ;
les figures 11a-d illustrent schématiquement des réalisation alternatives du toron à connecter.

L'homme de l'art appréciera que la présente invention puisse être appliquée essentiellement à tout type de connecteur, en particulier à tout type de micro-connecteur configuré pour des dispositifs implantables.

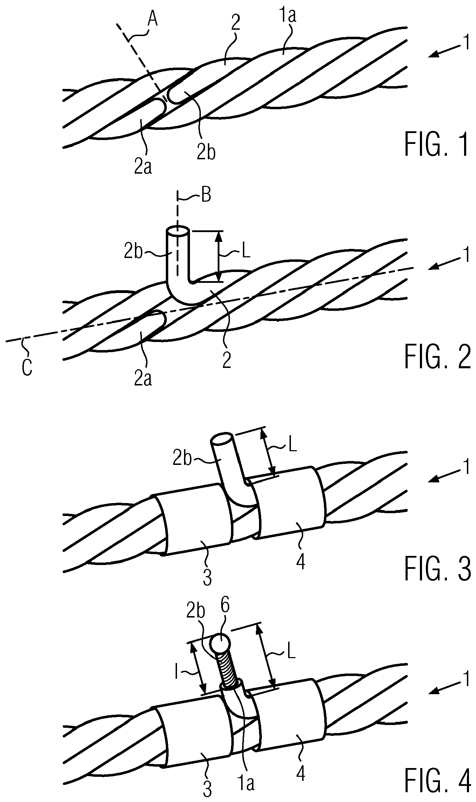

La figure 1 illustre un microcâble 1 multi-toron dont les dimensions sont de l'ordre du micron et qui est réalisé à partir de matériaux biocompatibles. Le microcâble 1 est formé de plusieurs torons, chaque toron étant pourvu d'une gaine 1a isolante en polymère. Dans l'étape du procédé représenté à la figure 1, un des torons a été découpé par laser, indiqué par le flèche A, de manière à ce que le toron 2 soit pourvu des deux extrémités libres 2a et 2b.

Dans une variante, un même toron pourrait être découpé en plusieurs endroits.

Dans un autre mode de réalisation, la découpe du toron 2 est réalisée au moyen d'un dispositif mécanique de type micro-couteau, pince, etc.

A l'étape illustrée par la figure 2, une portion de longueur L de l'extrémité libre 2b du toron 2 a été soulevée et partiellement extraite du reste du microcâble 1 de manière à ce que l'axe B de l'extrémité 2b du toron 2 soit sensiblement perpendiculaire à l'axe C de rotation du câble 1.

L'extrémité 2a, quant à elle, n'est pas soulevée par rapport au reste du microcâble 1.

A l'étape illustrée par la figure 3, un premier manchon isolant 3, en matériau polymère, est inséré, comme un anneau ou une bague, autour du câble 1 de manière à recouvrir l'extrémité 2a (qui devient ainsi non visible sur la figure 3).

Un deuxième manchon 4, aussi isolant et en matière polymère, est enfilé autour du câble 1 de manière à couvrir la portion de l'extrémité 2b qui n'a pas été extraite à l'étape précédente illustrée par la figure 2. Ainsi, ce deuxième manchon 4 permet ainsi de s'assurer que seule la portion de longueur L de l'extrémité 2b fait saillie sur la câble 1.

Dans un autre mode de réalisation, une colle polymère peut être utilisée pour maintenir la portion de l'extrémité 2b qui n'est pas prévue pour être soulevée et partiellement extraite du câble 1.

Dans le mode de réalisation illustré par la figure 3, le câble 1 est ainsi pourvu de deux manchons 3 et 4 telle que la portion de longueur L de l'extrémité 2b fait saillie entre les deux manchons 3 et 4.

La figure 4 illustre une étape où une partie I de la portion de longueur L de l'extrémité 2b est dénudée de son isolation en polymère 1a par ablation laser. De ce fait, la partie I de l'extrémité 2b correspond à la partie conductrice du toron 2. Une pointe sphérique 6 est formée au point le plus externe de l'extrémité 2b. Cette pointe sphérique 6, aussi appelée « bail tip » en anglais, permet de fusionner, en une sorte de balle ou boule, les multiples brins métalliques constituant le toron 2 ce qui évite que des brins individuels ne s'échappent et réduisent ainsi la fiabilité du contact électrique. La pointe sphérique 6 permet également de fournir une plus grande surface de contact, ce qui améliore davantage la fiabilité électrique du contact.

Dans un autre mode de réalisation, l'isolation de la longueur L de l'extrémité 2b est enlevée mécaniquement.

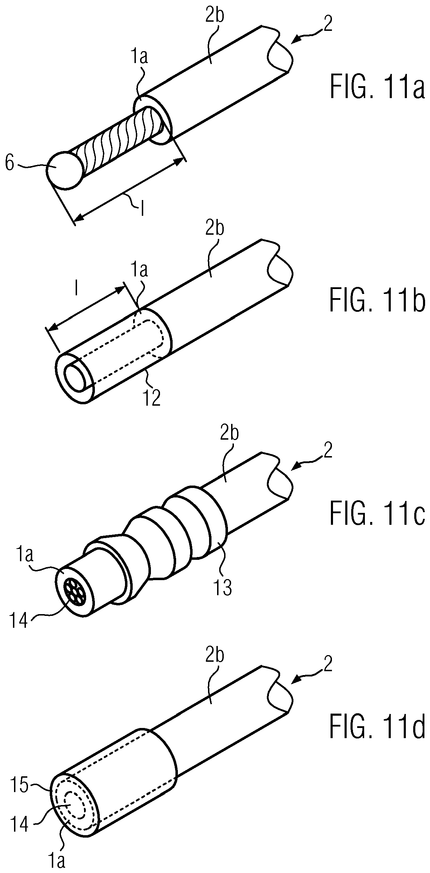

Des alternatives au retrait d'une portion I de la partie isolante 1a et à la formation d'une pointe sphérique 6 au bout de l'extrémité 2b sont décrits en figure 11.

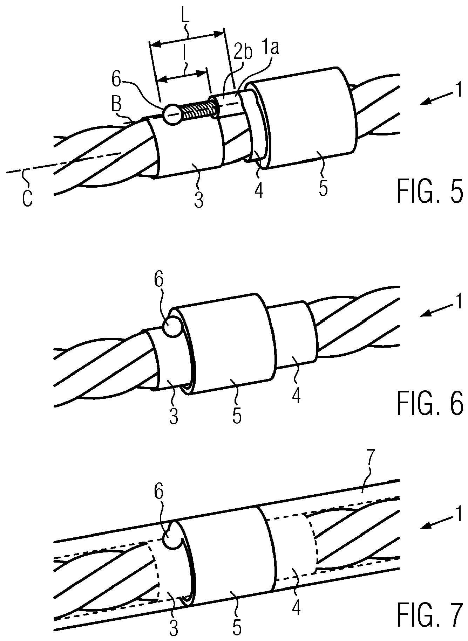

A la figure 5, une électrode cylindrique 5 est enfilée autour du microcâble 1 de manière à recouvrir partiellement le manchon 4 du côté opposé à celui du manchon 3. L'extrémité 2b est arrangée de manière à être posée sur le manchon 3 tel que l'axe B de la portion de longueur L de toron 2 soit parallèle à l'axe C du câble 1.

A l'étape illustrée par la figure 6, l'électrode cylindrique 5 est déplacée vers le manchon 3 de manière à recouvrir presque intégralement l'extrémité 2b. Ainsi, seule la pointe sphérique 6 de l'extrémité 2b n'est pas couverte par l'électrode 5. C'est la pointe sphérique 6 du toron 2, qui correspond à une partie conductrice du toron 2 car dépourvue d'isolation polymère 1a, qui est ensuite souder à l'électrode 5 au laser. De manière avantageuse, une telle soudure peut être réalisée avec un contrôle visuel.

De plus, le procédé de la présente invention permet d'éviter les difficultés liées à la présence de polymère dans une soudure laser, nuisant à sa qualité, en particulier à une échelle de l'ordre du micron.

Dans un autre mode de réalisation, l'électrode cylindrique 5 est sertie dans la position illustrée à la figure 6 avant l'étape de la soudure, afin d'assurer mécaniquement un maintien de sa position.

A la figure 7, un gainage 7 en polymère est réalisé afin de revêtir le câble multi-toron 1 et l'électrode 5 soudée au câble 1. Ce gainage peut par exemple être réalisé par la technique dite du « *reflow* », qui permet de faire fondre le polymère pour remplir les trous et espaces restants. Cette technique permet d'obtenir un produit fini isodiamétrique et d'ajouter une barrière isolante à l'assemblage.

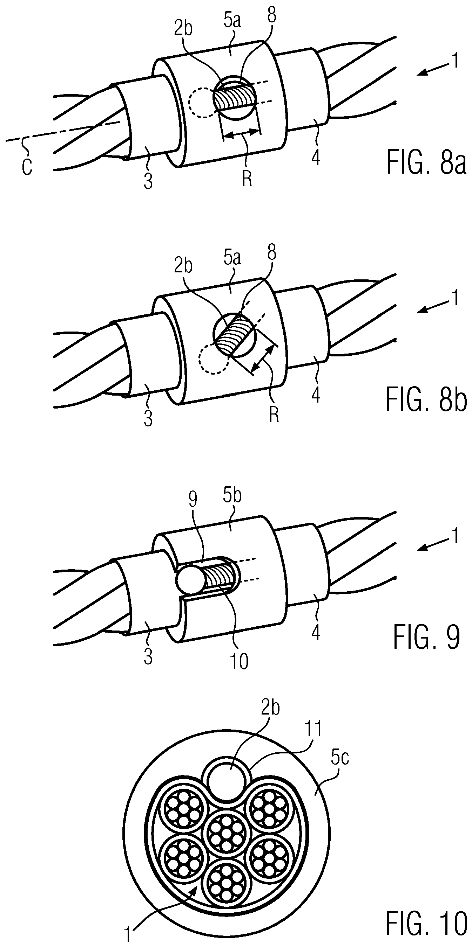

Les figures 8a, 8b, 9 et 10 illustrent des variantes de l'électrode 5.

Dans la variante illustrée aux figures 8a et 8b, l'électrode 5a est une bague avec un trou central 8. A la figure 8a, le toron 2b isolé du reste du câble 1 est aligné avec l'axe C du câble 1. A la figure 8b, le toron 2b est en reprise de rotation par rapport au câble 1. Toutefois, dans les deux variantes, la soudure est réalisée au niveau du trou central 8 qui est arrangé au-dessus d'une portion R du toron 2b.

A la figure 9, l'électrode 5b est une bague avec une fente en «U» 9, aussi dite avec chargement frontal. Dans un autre mode de réalisation, la fente pourrait être en «V», en arc, etc. Dans ce mode de réalisation, l'extrémité du toron 2b est arrangée de manière à être alignée entre les côtés longitudinaux 9 et 10 de la fente 9. La soudure est ainsi réalisée au niveau de la fente 9 entre le toron 2b et la bague 5b.

Au mode de réalisation de la figure 10, l'électrode 5c est une bague comprenant une cavité interne 11. La cavité interne 11 de la bague 5c est adaptée au diamètre de l'extrémité du toron 2b soulevé. L'extrémité du toron 2b est ainsi partiellement logée dans la cavité interne 11. Cette cavité interne 11 permet de laisser l'espace nécessaire à l'extrémité du toron 2b à souder et permet ainsi d'améliorer la surface de contact électrique.

La figure 11 illustre des alternatives à la formation d'une pointe sphérique 6 pour réaliser le contact électrique avec l'élément conducteur 5, tel que décrit ci-dessus et représenté en figure 11a.

Ainsi, dans la variante illustrée par la figure 11b, l'extrémité 2b du toron 2 a été dénudée sur une longueur I, puis un hypotube métallique creux 12 a été inséré autour de la longueur I dénudée du toron 2. La surface de contact entre la partie I dénudée et l'hypotube métallique permet d'assurer le contact électrique.

A la variante illustrée en figure 11c, l'extrémité 2b du toron 2 n'est pas dénudée et donc comprend encore sa gaine isolante 1a. Un hypotube métallique creux 13 a été inséré autour de l'extrémité 2b et serti de manière à ce que le sertissage de hypotube 13 soit perforant pour réaliser un contact électrique avec la partie conductrice 14 du toron 2.

La variante de la figure 11d représente un toron 2 dont l'extrémité 2b n'est pas dénudée et donc qui comprend encore sa gaine isolante 1a. Un hypotube métallique creux 13 a été inséré autour de l'extrémité 2b et soudé à l'extrémité 2b, en particulier souder à la partie conductrice 14 du toron 2.

Dans encore une autre variante qui n'est pas illustrée, la soudure peut être réalisée au niveau des deux extrémités libres 2a, 2b du même toron 2 afin de garantir une redondance de la liaison.

Dans encore une autre variante qui n'est pas illustrée, deux torons en opposition peuvent être découpés, soulevés et partiellement extraits du reste du câble et soudés des deux côtés.

Enfin, dans une autre variante non-représentée, la séparation des brins d'un même câble en un nombre n de sous-câbles ou fils permet de faire de la redondance de soudure et ainsi d'améliorer la fiabilité du contact électrique avec l'élément conducteur par augmentation du nombre de soudures. Notamment dans le cas où une des soudures se casse, les n-1 soudures permettent d'assurer le contact électrique. Ainsi, la redondance de soudure améliore la robustesse du contact électrique.

Les modes de réalisation et les variantes discutées précédemment peuvent être combinés pour former davantage de variantes de mode de réalisation avantageux de la présente invention.

## Revendications

1. Procédé pour connecter un toron d'un câble multi-toron à une électrode d'un dispositif médical implantable, comprenant les étapes de :
a) Découper un toron (2) du câble multi-toron (1) afin de former deux extrémités libres (2a, 2b) au niveau de la coupe (A) du toron (1);
b) Soulever au moins une des extrémités libres (2b) par rapport au reste du câble multi-toron (1);
c) Dénuder au moins partiellement l'extrémité du toron (2b) soulevée;
d) Placer une électrode (5) autour du câble multi-toron (1) de manière à recouvrir partiellement l'extrémité du toron soulevée et dénudée (2b);
e) Connecter au moins une portion (6) de l'extrémité dénudée (2b) du toron (2) à l'électrode (5).

2. Le procédé selon la revendication 1, où l'étape b) comprend : recouvrir l'autre des extrémités libres (2a) du toron (2) en introduisant un manchon isolant (3) autour du câble (1).

3. Le procédé selon la revendication 2, où l'étape b) comprend également la pose d'un deuxième manchon (4) ou/et d'une colle polymère autour du câble multi-toron (1) afin de contrôler la longueur (L) de l'extrémité du toron (2b) soulevée par rapport au reste du câble multitoron (1).

4. Le procédé selon l'une des revendications précédentes, où l'étape a) et c) comprennent une découpe ou/et ablation laser ou l'utilisation d'un dispositif mécanique de découpe.

5. Procédé pour connecter un toron d'un câble multi-toron à une électrode d'un dispositif médical implantable, comprenant les étapes de :
a) Découper un toron (2) du câble multi-toron (1) afin de former deux extrémités libres (2a, 2b) au niveau de la coupe (A) du toron (1);
b) Soulever au moins une des extrémités libres (2b) par rapport au reste du câble multitoron (1);
c) Placer un hypotube métallique autour de l'extrémité du toron (2b) soulevée de manière à ce que l'hypotube (12, 13, 14) soit électriquement connecté à l'extrémité du toron (2b);
d) Placer une électrode (5) autour du câble multi-toron (1) de manière à recouvrir partiellement l'hypotube métallique (12, 13, 14);
e) Connecter au moins une portion de l'hypotube métallique (12, 13, 14) à l'électrode (5).

6. Le procédé selon l'une des revendications précédentes, où la connexion à l'étape e) est réalisée par soudure laser, par sertissage ou par soudure électrique.

7. Le procédé selon l'une des revendications précédentes, comprenant une étape f) pour poser un gainage (7) en polymère afin de revêtir le câble multi-toron (1) et l'électrode soudée au câble (1).

8. Le procédé selon l'une des revendications précédentes, où l'électrode (5) est une électrode en forme de bague (5a, 5b) telle que la bague (5a, 5b) comprend un trou central (8) ou une fente (9) configuré pour réaliser la soudure à l'étape e).

9. Le procédé selon l'une des revendications précédentes, où l'électrode (5) est une électrode en forme de bague (5c) telle que la bague (5c) comprend une cavité interne (11) adaptée à la dimension de l'extrémité (2b) du toron soulevé.

10. Le procédé selon l'une des revendications précédentes, dans lequel le diamètre du toron découpé (2) - en prenant en considération la partie conductrice et la partie isolante du toron - est compris entre 10 µm et 200µm; le diamètre du microcâble multi-toron, c'est-à-dire le diamètre du câble entier, est inférieur à 2 French (soit 0,66mm) et est réalisé à partir de matériaux biocompatibles.

11. Microcâble multi-toron comprenant au moins une électrode (5) d'un dispositif médical implantable tel qu'au moins une extrémité (2b) d'un toron (2) du microcâble (1) est partiellement dénudée de manière à ce que la portion partiellement dénudée du toron est connectée, en particulier par soudure, sertissage ou soudure électrique, à l'électrode (5), en particulier réalisé selon le procédé d'au moins une des revendications 1 à 5 ou 7 à 10.

12. Microcâble multi-toron comprenant au moins une électrode (5) d'un dispositif médical implantable et un hypotube métallique (12, 13, 14) tel qu'au moins une extrémité (2b) d'un toron (2) du microcâble (1) est au moins partiellement en contact électrique avec l'hypotube, de manière à ce que l'hypotube est connecté, en particulier par soudure, sertissage ou soudure électrique, à l'électrode (5), en particulier réalisé selon le procédé d'au moins une des revendications 6 à 10.

13. Le microcâble multi-toron selon la revendication 11, comprenant un manchon isolant (3) positionné autour du câble (1) et sur lequel l'extrémité (2b) du toron (2) est positionnée.

14. Le microcâble multi-toron selon l'une des revendications 11 à 13, dont l'électrode (5) est une électrode en forme de bague (5a, 5b) telle que la bague (5a, 5b) comprend un trou central (8) ou une fente (9) configuré pour réaliser la soudure avec la portion (6) d'une extrémité (2b) du toron (2).

15. Le microcâble multi-toron selon l'une des revendications 11 à 14, dont l'électrode (5) est une électrode en forme de bague (5c) telle que la bague (5c) comprend une cavité interne (11) adaptée à la dimension de l'extrémité (2b) du toron (2) arrangée partiellement au-dessus du manchon isolant (3).

16. Microcâble multi-toron selon l'une des revendications 11 à 15, dont le diamètre d'un toron - en prenant en considération la partie conductrice et la partie isolante du toron - est compris entre 10µm et 200µm; le diamètre du microcâble multi-toron, c'est-à-dire le diamètre du câble entier, est inférieur à 2 French (soit 0,66mm) et est réalisé à partir de matériaux biocompatibles.
